# EUROPEAN PATENT APPLICATION

(11) **EP 4 847 125 A1**
(43) Date of publication of application: **02.12.2026**
(21) Application number: 25745320.9
(22) Date of filing: 22.01.2025
(51) Int. Cl.: B01J 23/72, B01J 37/00, B01J 37/08, B01J 21/08, B01J 37/03, B01J 35/37, C07C 29/141, C07C 31/20

(54) **COPPER SILICATE-BASED CATALYST AND PREPARATION METHOD THEREFOR**

(30) Priority: 23.01.2024 KR 20240009934
(71) Applicant: LG CHEM, LTD., Seoul 07336 (KR)
(72) Inventor: SUH, Myungji, Daejeon 34122 (KR); KIM, Tae Yun, Daejeon 34122 (KR); EOM, Sungshik, Daejeon 34122 (KR)
(74) Representative: Goddar, Heinz J.
(86) International application number: PCT/KR2025/001228
(87) International publication number: WO 2025/159495

(57) **Abstract**

A copper silicate-based catalyst according to an exemplary embodiment of the present application contains copper and silica, wherein the weight ratio of Cu : Si in the copper silicate-based catalyst is 30 : 70 to 70 : 30, and the Cu 2p binding energy of the copper silicate-based catalyst determined by XPS (X-ray photoelectron spectroscopy) analysis is 932 eV to 934 eV.

## Description

### [Technical Field]

This application claims the benefit of Korean Patent Application No. 10-2024-0009934 filed in the Korean Intellectual Property Office on January 23, 2024, the entire contents of which are incorporated herein by reference.

This application relates to a copper silicate-based catalyst and a method for preparing the same.

### [Background Art]

Neopentyl glycol (NPG) is a white crystalline substance with a melting point of 130 °C or higher. It is used as an important intermediate for various synthetic resins and is also widely used industrially as a raw material for various plastic powder paints, synthetic lubricants, plasticizers, surfactants, fiber processing agents, etc.

The NPG is generally prepared by preparing hydroxypivaldehyde (HPA) through an aldol condensation reaction of isobutyraldehyde and formaldehyde, and then reacting the HPA with hydrogen in the presence of a catalyst.

Conventionally, the HPA has been subjected to hydrogenation reaction using a slurry-type Ni-based catalyst. In this case, crude NPG, which is a hydrogenation reaction product, contains 2,2,4-trimethyl-1,3-pentanediol (TMPD), hydroxypivalic acid NPG ester (HPNE), etc. Since TMPD and HPNE have boiling points very similar to that of NPG, they cannot be separated by simple distillation. Since HPNE is unstable when distilling the reaction mixture and leads to a decrease in the yield of NPG, it is commercially converted to NPG by saponification reaction by adding sodium hydroxide. However, since sodium salts of HPA or other organic acids produced by the saponification reaction promote the decomposition reaction of NPG at high temperatures of 140 °C or higher, the distillation process is constrained. Furthermore, it is impossible to remove TMPD which has not been converted to a non-volatile sodium salt during the saponification reaction.

Therefore, efforts for producing NPG at a high yield in an economical way are being made continuously in the art.

### [Disclosure]

### [Technical Problem]

The present application provides a copper silicate-based catalyst and a method for preparing the same.

### [Technical Solution]

An exemplary embodiment of the present application provides a copper silicate-based catalyst containing copper and silica, wherein
a weight ratio of Cu : Si in the copper silicate-based catalyst is 30 : 70 to 70 : 30, and
a Cu 2p binding energy of the copper silicate-based catalyst determined by XPS (X-ray photoelectron spectroscopy) analysis is 932 eV to 934 eV.

In addition, another exemplary embodiment of the present application provides a method for preparing a copper silicate-based catalyst, which comprises:
a step of preparing a coprecipitated slurry by adding a silica sol and an alkaline precipitant to an aqueous solution containing a copper precursor;
a step of aging the coprecipitated slurry at 80 °C to 90 °C for 3 hours or longer, and then obtaining a dried product by filtering and drying the same;
a step of mixing the dried product with a fiber-based binder and an organic compound, and then preparing a catalyst precursor by molding the same; and
a step of drying the catalyst precursor, and then sintering the same at 400 °C to 650 °C, wherein
a content of the fiber-based binder is 0.1 wt% or more and less than 15 wt% based on the total weight of the dried product.

In addition, another exemplary embodiment of the present application provides a method for preparing neopentyl glycol, which comprises a step of conducting hydrogenation reaction by adding a hydroxypivaldehyde (HPA) solution and hydrogen to a hydrogenation reactor, wherein
the hydrogenation reactor contains the copper silicate-based catalyst.

### [Advantageous Effects]

According to an exemplary embodiment of the present application, a copper silicate-based catalyst with improved stability can be prepared.

In particular, according to an exemplary embodiment of the present application, the 2p binding energy, Cu dispersibility, Cu cubic crystallite size and acid strength characteristics of the catalyst can be optimized by controlling the aging condition of a coprecipitated slurry and the sintering condition of a catalyst precursor. Through this, the decline in the strength, reactivity, etc. of the catalyst may be prevented even under the high-temperature, high-pressure reaction condition for preparation of neopentyl glycol with a high yield.

Accordingly, when the copper silicate-based catalyst according to an exemplary embodiment of the present application is used to prepare neopentyl glycol, the preparation process can be maintained stably and, therefore, economical efficiency can be enhanced.

### [Brief Description of Drawings]

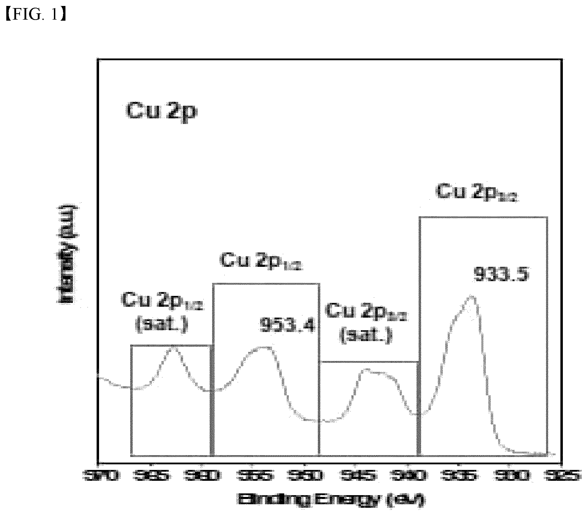

FIG. 1 shows the XPS spectrum of a copper silicate-based catalyst of Example 1 according to the present application.

### [Best Mode]

Hereinafter, the present specification is described in more detail.

In the present specification, when a member is described to be located "on" another member, it comprises not only the case where the two members are in contact with each other but also the case where another member is present between the two members.

In the present specification, when a part is described to "comprise" a certain component, it means that it may comprise other components and does not exclude other components, unless the context specifically states otherwise.

As stated above, efforts for producing NPG at a high yield in an economical way are being made continuously in the art.

In particular, neopentyl glycol is by hydrogenation of HPA (hydroxypivaldehyde) in the presence of a catalyst under high-temperature (160 °C or higher) and high-pressure (35 bar or higher) conditions. Since the strength and reactivity of the catalyst may decline under the high-temperature, high-pressure reaction conditions due to the elution of catalyst components, the hydrogenation process may not be operated stably.

Therefore, the present application is directed to providing a copper silicate-based catalyst with superior stability, which can be applied to the preparation process of neopentyl glycol, and a method for preparing the same.

The copper silicate-based catalyst according to an exemplary embodiment of the present application contains copper and silica, wherein the weight ratio of Cu : Si in the copper silicate-based catalyst is 30 : 70 to 70 : 30, and the Cu 2p binding energy of the copper silicate-based catalyst determined by XPS (X-ray photoelectron spectroscopy) analysis is 932 eV to 934 eV.

In an exemplary embodiment of the present application, the weight ratio of Cu : Si in the copper silicate-based catalyst may be 30 : 70 to 70 : 30, 30 : 70 to 50 : 50, or 35 : 65 to 50 : 50. The weight ratio of Cu : Si in the copper silicate-based catalyst is an indicator of the content of Cu, which is the active component of the catalyst. If the weight ratio of Cu : Si is outside the above ranges, the activity of the catalyst declines undesirably.

In an exemplary embodiment of the present application, the Cu 2p binding energy of the copper silicate-based catalyst determined by XPS (X-ray photoelectron spectroscopy) analysis may be 932 eV to 934 eV, 933 eV to 934 eV, or 933.6 eV to 933.8 eV.

In an exemplary embodiment of the present application, the Cu 2p binding energy of the copper silicate-based catalyst determined by XPS (X-ray photoelectron spectroscopy) analysis means a main peak appearing as a high peak within the binding energy range of 928.0 eV to 939.0 eV in the Cu 2p spectrum due to rapid increase of intensity. The Cu 2p binding energy of the copper silicate-based catalyst as a result of XPS (X-ray photoelectron spectroscopy) analysis being 932 eV to 934 eV is the characteristic of catalyst according to an exemplary embodiment of the present application. Outside the above range, the activity of the catalyst declines undesirably.

A specific method for measuring the Cu 2p binding energy of the copper silicate-based catalyst as a result of XPS (X-ray photoelectron spectroscopy) analysis is described in the examples given below.

In an exemplary embodiment of the present application, the Cu dispersibility of the copper silicate-based catalyst determined by N₂O pulse chemisorption analysis may be 0.5% to 3%, 0.7% to 2.8%, or 0.9% to 2.4%, and the Cu cubic crystallite size may be 200 nm or smaller, 10 nm to 150 nm, or 39 nm to 97 nm.

In an exemplary embodiment of the present application, the Cu dispersibility and the Cu cubic crystallite size indicate the dispersibility of Cu, which is the active component of the catalyst. If the Cu dispersibility and the Cu cubic crystallite size are outside the above ranges, the activity of the catalyst declines undesirably.

Specific methods for measuring the Cu dispersibility and Cu cubic crystallite size of the copper silicate-based catalyst determined by N₂O pulse chemisorption analysis are described in the examples given below.

In an exemplary embodiment of the present application, upon NH₃-TPD (temperature-programmed desorption) analysis of the copper silicate-based catalyst, peaks may be present in the weak acid site region of 100 °C or higher and lower than 400 °C and the strong acid site region of 400 °C or higher and 700 °C or lower, and the total acid amount may be 0.03 mmol to 2.0 mmol, 0.1 mmol to 1.9 mmol, 0.5 mmol to 1.8 mmol, or 1.342 mmol to 1.784 mmol.

Specific methods for measuring the weak acid sites, strong acid sites and total acid amount of the copper silicate-based catalyst by NH₃-TPD (temperature-programmed desorption) analysis are described in the examples given below.

In an exemplary embodiment of the present application, the NH₃-TPD analysis investigates the characteristics of a catalyst such as acid strength and acid sites by adsorbing NH₃, which is a base, to the catalyst. If the adsorbed base is desorbed at low temperature ranges of 100 °C or higher and lower than 400 °C, it means that the acid strength is weak. And, a desorption peak appearing at high temperature ranges of 400 °C or higher and 700 °C or lower means strong acid strength. The acid amount of weak acid sites and the acid amount of strong acid sites of the catalyst vary depending on the various characteristics of the catalyst, for example, the content of active components, raw material, preparation method, heat treatment method, etc. Accordingly, if the acid amount of weak acid sites and the acid amount of strong acid sites are outside the above ranges, the activity of the catalyst declines undesirably because of the change in the surface characteristics of the catalyst.

In an exemplary embodiment of the present application, the copper silicate-based catalyst may have a crushing strength of 40 N or higher, specifically 42 N to 150 N, or 45 N to 70 N. If the crushing strength of the copper silicate-based catalyst is lower than 40 N, the catalyst may be damaged easily when it is put in a reactor, and a differential pressure may be formed undesirably in the reactor due to damage within a relatively short period.

A specific method for measuring the crushing strength of the copper silicate-based catalyst is described in the examples given below.

In an exemplary embodiment of the present application, the copper silicate-based catalyst may be a catalyst extrusion-molded into a cylindrical shape with a diameter of 2 mm to 6 mm and a height of 2 mm to 10 mm, or a catalyst extrusion-molded into a cylindrical shape with a diameter of 3 mm to 5 mm and a height of 3 mm to 9 mm. If the diameter of the cylindrical catalyst exceeds 6 mm or its height exceeds 10 mm, catalytic activity may decrease undesirably due to decreased surface area. In addition, if the diameter of the cylindrical catalyst is smaller than 2 mm or its height is smaller than 2 mm, crushing and drifting may occur undesirably under the high-temperature, high-pressure reaction condition for preparation of neopentyl glycol.

The molding of the catalyst is important for physical durability and flow of reactants in the reactor. Although a powder catalyst may be advantageous in terms of catalytic activity because of large surface area due to small particle size, the flow of reactants in a commercial-scale fixed-bed reactor is limited and drifting or differential pressure may occur. For this reason, it is desirable to mold the catalyst by extrusion, compression, coating, etc. The pores formed by the molded catalyst in the fixed-bed reactor provide the effect of reducing differential pressure caused by reactants supplied at a high flow rate for commercial productivity. In particular, since the extrusion-molded catalyst has the advantage of high durability due to high strength, the possibility of drifting or differential pressure caused by wearing, crushing, etc. during long-term use of the catalyst may be decreased.

In an exemplary embodiment of the present application, the copper silicate-based catalyst may be for preparation of neopentyl glycol.

In addition, a method for preparing a copper silicate-based catalyst according to an exemplary embodiment of the present application comprises: a step of preparing a coprecipitated slurry by adding a silica sol and an alkaline precipitant to an aqueous solution containing a copper precursor; a step of aging the coprecipitated slurry at 80 °C to 90 °C for 3 hours or longer, and then obtaining a dried product by filtering and drying the same; a step of mixing the dried product with a fiber-based binder and an organic compound, and then preparing a catalyst precursor by molding the same; and a step of drying the catalyst precursor, and then sintering the same at 400 °C to 650 °C.

The method for preparing a copper silicate-based catalyst according to an exemplary embodiment of the present application comprises a step of preparing a coprecipitated slurry by adding a silica sol and an alkaline precipitant to an aqueous solution containing a copper precursor.

In an exemplary embodiment of the present application, the copper precursor may be Cu(NO₃)₂·3H₂O, Cu(CO₃)₂·Cu(OH)₂, CuCl₂·2H₂O, etc., although not being limited thereto.

In an exemplary embodiment of the present application, the alkaline precipitant may be an alkali metal hydroxide, an alkali metal carbonate, an alkali metal bicarbonate, a mixture thereof, etc. More specifically, the alkaline precipitant may be one or more of NH₄OH, (NH₄)₂CO₃, NH₄HCO₃, CH₄N₂O, NaOH and Na₂CO₃, although not being limited thereto.

When adding the alkaline precipitant, the temperature increased by heat of neutralization may be lowered to room temperature using cooling water, etc.

The method for preparing a copper silicate-based catalyst according to an exemplary embodiment of the present application comprises a step of aging the coprecipitated slurry at 80 °C to 90 °C for 3 hours or longer, and then obtaining a dried product by filtering and drying the same.

The aging may be performed at 80 °C to 90 °C for 3 hours or longer, or at 80 °C to 90 °C for 3 hours to 8 hours.

Bonding between the copper precursor and the silica is formed by the aging. The copper precursor does not remain unreacted when both the aging temperature and time described above are satisfied. If the copper precursor remains unreacted, the activity of the catalyst may decrease undesirably.

After the aging, the coprecipitated slurry may be filtered by a method known in the art without special limitation.

In an exemplary embodiment of the present application, the filtered coprecipitated slurry may be dried at 60 °C to 120 °C for 5 hours to 72 hours, or at 70 °C to 110 °C for 7 hours to 48 hours.

The method for preparing a copper silicate-based catalyst according to an exemplary embodiment of the present application comprises a step of mixing the dried product with a fiber-based binder and an organic compound, and then preparing a catalyst precursor by molding the same.

In an exemplary embodiment of the present application, the fiber-based binder may comprise one or more of glass fiber, carbon fiber, aramid fiber, alumina fiber, aluminum silicate fiber, silicon carbide fiber and boron fiber.

In an exemplary embodiment of the present application, the content of the fiber-based binder may be 0.1 wt% or more and less than 15 wt%, 1 wt% to 10 wt%, or 3 wt% to 7 wt%, based on the total weight of the dried product. If the content of the fiber-based binder is less than 0.1 wt% based on the total weight of the dried product, physical stability may worsen undesirably under the high-temperature, high-pressure reaction condition due to significantly decreased strength of the catalyst. And, if the content of the fiber-based binder is 15 wt% or more, productivity may decrease during the preparation of the catalyst because of poor flowability during the extrusion molding of the catalyst and the strength of the catalyst may also decline because of poor compressibility.

In an exemplary embodiment of the present application, the content of the organic compound may be 0.1 wt% to 50 wt%, 1 wt% to 40 wt%, or 10 wt% to 30 wt%, based on the total weight of the dried product. The organic compound serves as a lubricant during the molding of the catalyst. If the content of the organic compound is less than 0.1 wt% based on the total weight of the dried product, molding may be impossible. And, if it exceeds 50 wt%, the strength or activity of the catalyst may decline undesirably.

In an exemplary embodiment of the present application, the organic compound may comprise one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

The method for preparing a copper silicate-based catalyst according to an exemplary embodiment of the present application comprises a step of drying the catalyst precursor, and then sintering the same at 400 °C to 650 °C.

In an exemplary embodiment of the present application, the catalyst precursor may be dried at 60 °C to 120 °C for 5 hours to 24 hours, or at 70 °C to 110 °C for 7 hours to 20 hours.

In an exemplary embodiment of the present application, the dried catalyst precursor may be sintered at 400 °C to 650 °C for 5 hours to 24 hours, or at 450 °C to 600 °C for 6 hours to 20 hours.

If the drying condition and sintering condition are outside the above ranges, catalytic activity may decline undesirably due to change in the crystallinity of the catalyst.

In addition, another exemplary embodiment of the present application provides a method for preparing neopentyl glycol, which comprises a step of adding a hydroxypivaldehyde (HPA) solution and hydrogen to a hydrogenation reactor and conducting hydrogenation reaction, wherein the hydrogenation reactor contains the copper silicate-based catalyst.

In an exemplary embodiment of the present application, a method known in the art may be used for the preparation of neopentyl glycol, except for using the copper silicate-based catalyst.

For example, the hydrogenation reactor may be a fixed-bed reactor (FBR) charged with the copper silicate-based catalyst. In this case, it is not necessary to separate the reaction product from the catalyst, the operation is stable and economical because reaction temperature and reaction pressure can be lowered, the replacement of the catalyst is easy, and the investment cost can be reduced greatly because the reactor size can be decreased.

In addition, the hydroxypivaldehyde solution may contain 65 wt% or less of hydroxypivaldehyde, 10 wt% or less of neopentyl glycol, 15 wt% to 35 wt% of an alcohol, and 15 wt% or less of water. In this case, the production of byproducts may be suppressed since the heat of reaction can be minimized without sacrificing reactivity.

The hydrogenation reaction may be performed at 100 °C to 250 °C, 100 °C to 200 °C, or 100 °C to 180 °C.

According to an exemplary embodiment of the present application, a copper silicate-based catalyst with improved stability may be prepared.

In particular, according to an exemplary embodiment of the present application, by controlling the aging condition of the coprecipitated slurry, the content of the fiber-based binder, and the sintering condition of the catalyst precursor, the 2p binding energy, Cu dispersibility, Cu cubic crystallite size and acid strength characteristics of the catalyst can be optimized and, accordingly, the decline in the strength, reactivity, etc. of the catalyst can be prevented even under the high-temperature, high-pressure reaction condition for preparation of neopentyl glycol with a high yield.

Accordingly, when the copper silicate-based catalyst according to an exemplary embodiment of the present application is used to prepare neopentyl glycol, the preparation process can be maintained stably and, therefore, economical efficiency can be enhanced.

### [Mode for Invention]

Hereinafter, the present application will be described in detail through specific examples. However, the examples according to the present application can be changed into various other forms, and it should not be interpreted that the scope of the present application is limited by the examples described below. The examples of the present application are provided to describe the present application more completely to those having ordinary knowledge in the art.

### <Examples>

### <Example 1>

A copper precursor (Cu(NO₃)₂·3H₂O) aqueous solution was prepared in a double jacket reactor, and silica sol was added such that the weight ratio of Cu and Si was Cu : Si = 30 : 70. After adding a NaOH aqueous solution, a coprecipitate was prepared by increasing the temperature of the reactor and performing aging at 80 °C for 3 hours. After filtering the coprecipitate and washing with distilled water, a wet cake obtained thereby was dried in an oven. The dried product was crushed to obtain a catalyst intermediate powder.

After mixing 25 wt% of an organic compound (20 wt% of isopropyl alcohol and 5 wt% of glycerin), 5 wt% of a fiber-based binder (ceramic wool, aluminum silicate) and distilled water, based on the total weight of the catalyst intermediate powder, the catalyst intermediate was extrusion-molded into a cylindrical shape with a diameter of 3 mm to 5 mm and a height of 3 mm to 9 mm using an extruder.

Finally, a catalyst for preparation of neopentyl glycol was prepared by sintering the extrusion-molded catalyst intermediate at 550 °C for 8 hours.

### <Example 2>

A catalyst for preparation of neopentyl glycol was prepared in the same manner as in Example 1, except that the coprecipitate was prepared such that the weight ratio of Cu and Si was Cu : Si = 40 : 60.

### <Example 3>

A catalyst for preparation of neopentyl glycol was prepared in the same manner as in Example 1, except that the coprecipitate was prepared such that the weight ratio of Cu and Si was 50 : 50.

### <Comparative Example 1>

A catalyst for preparation of neopentyl glycol was prepared in the same manner as in Example 1, except that the aging was performed at 65 °C for 3 hours rather than at 80 °C for 3 hours.

### <Comparative Example 2>

A catalyst for preparation of neopentyl glycol was prepared in the same manner as in Example 2, except that the aging was performed at 65 °C for 3 hours rather than at 80 °C for 3 hours.

### <Comparative Example 3>

A catalyst for preparation of neopentyl glycol was prepared in the same manner as in Example 1, except that the sintering was performed at 700 °C for 8 hours rather than at 550 °C for 8 hours.

### <Comparative Example 4>

A catalyst for preparation of neopentyl glycol was prepared in the same manner as in Example 2, except that the sintering was performed at 700 °C for 8 hours rather than at 550 °C for 8 hours.

### <Comparative Example 5>

A catalyst for preparation of neopentyl glycol was prepared in the same manner as in Example 1, except that 30 wt% of an organic compound (20 wt% of isopropyl alcohol and 10 wt% of glycerin) and 15 wt% of a fiber-based binder were used in the extrusion molding step instead of the 25 wt% of the organic compound and 5 wt% of the fiber-based binder.

### <Comparative Example 6>

A catalyst for preparation of neopentyl glycol was prepared in the same manner as in Example 1, except that the organic compound and the fiber-based binder were not used in the extrusion molding step.

### <Comparative Example 7>

A catalyst for preparation of neopentyl glycol was prepared in the same manner as in Example 1, except that the fiber-based binder was not used in the extrusion molding step.

### <Comparative Example 8>

A catalyst for preparation of neopentyl glycol was prepared in the same manner as in Example 1, except that the organic compound was not used. However, extrusion was impossible because lubrication was not performed smoothly in the extruder during the extrusion molding step. As a result, a catalyst could not be prepared.

### <Test Example>

In the presence of the catalyst prepared in the examples or comparative examples, hydrogenation reaction was performed at 160 °C and 35 bar for 1 hour using an HPA solution consisting of 65 wt% of HPA, 2 wt% of NPG, 25 wt% of 2-ethylhexanol, 5 wt% of H₂O and 3 wt% of a high-boiling-point material, and catalytic activity was measured by measuring the amount of hydrogen consumed. The result is shown in Table 1. The catalytic activity was represented relative to the result of Example 1 as 100.

In addition, the crushing strength, Cu dispersibility, and Cu cubic crystallite size of the catalysts of the examples and comparative examples were evaluated. The result is shown in Table 1.

In addition, the Cu 2p binding energy and NH₃-TPD peak positions of the catalysts of the examples and comparative examples were evaluated by XPS analysis. The result is shown in Table 2.

In addition, the XPS spectrum of the copper silicate-based catalyst of Example 1 is shown in FIG. 1.

The measurement methods of the evaluation results shown in Tables 1 and 2 are as follows.

### <Crushing strength>

Crushing strength was measured using SHIMPO's FGN-50B. More specifically, after placing the catalyst extrusion-molded to a cylindrical shape on a strength tester holder such that the side surface faces the bottom, the maximum pressure value was measured as the measuring unit compresses the catalyst downward from the top. Measurement was made for 20 catalysts and the result was averaged.

For Example 1, although it was intended to measure the crushing strength of the catalyst intermediate powder without conducting the extrusion molding step, the measurement was impossible with the method using SHIMPO's FGN-50B because the particle size of the powder catalyst was small as about 100 µm. Thus, the crushing strength of the powder catalyst was evaluated as 0.

### <Cu dispersibility and Cu cubic crystallite size>

Cu dispersibility and Cu cubic crystallite size were measured by N₂O pulse chemisorption analysis.

More specifically, using Micromeritics' AutoChem, about 0.2 g of a catalyst sample was filled in a U-shaped tube and pre-treated at 250 °C for 2 hours under hydrogen atmosphere. Then, after pulse-injecting N₂O at 60 °C using a loop of a predetermined volume, the surface area, dispersibility and cubic crystallite size of Cu were calculated from the accumulated consumption amount of N₂O and the Cu wt% of the catalyst.

### <Cu 2p binding energy>

Cu 2p binding energy was measured by XPS (X-ray photoelectron spectroscopy) analysis.

More specifically, after preparing a circular pellet sample with a diameter of 2 mm and a thickness of 1 mm by crushing the catalyst, the sample was measured using a monochromatic Al k-alpha X-ray α (1486.6 eV) with a size of 400 µm X 800 µm. After obtaining a first XPS spectrum (before correction) by XPS (X-ray photoelectron spectroscopy) under the condition of vacuum atmosphere, a measurement range of 925 eV to 970 eV, and a pass energy of 50 eV, a second XPS spectrum (after correction) was obtained by correcting the peaks of the XPS spectrum. The Cu 2p binding energy is a value derived from the second XPS spectrum.

### <Weak acid sites, strong acid sites and total acid amount>

Weak acid sites, strong acid sites and total acid amount were measured by NH₃-TPD (temperature-programmed desorption) analysis.

More specifically, using Micromeritics' AutoChem, about 0.1 g of the catalyst sample was filled in a U-shaped tube and pre-treated at 200 °C for 1 hour under helium atmosphere. Then, after adsorbing NH₃ at 100 °C for 1 hour, desorption value was measured while raising temperature to 800 °C under helium atmosphere at a rate of 5 °C/min.

**[Table 1]**

| | Catalytic activity | Crushing strength (N) | N₂O pulse chemisorption | |
|---|---|---|---|---|
| | | | Cu dispersibility (%) | Cu cubic crystalline size (nm) |
| Example 1 | 100 | 45 | 2.4 | 39 |
| Example 2 | 102 | 51 | 1.4 | 60 |
| Example 3 | 106 | 70 | 0.9 | 97 |
| Comparative Example 1 | 86 | 63 | 1.8 | 67 |
| Comparative Example 2 | 87 | 72 | 1.3 | 88 |
| Comparative Example 3 | 0 | 105 | 0.4 | 237 |
| Comparative Example 4 | 0 | 91 | 0.4 | 230 |
| Comparative Example 5 | 75 | 49 | 1.4 | 59 |
| Comparative Example 6 | 75 | 20 | 0.4 | 152 |
| Comparative Example 7 | 63 | 35 | 0.4 | 178 |

**[Table 2]**

| | XPS | NH₃-TPD | | |
|---|---|---|---|---|
| | Cu 2p binding energy | Weak acid sites (%) | Strong acid sites (%) | Total acid amount (mmol) |
| Example 1 | 933.6 | 71 | 29 | 1.784 |
| Example 2 | 933.8 | 71 | 29 | 1.600 |
| Example 3 | 933.6 | 87 | 13 | 1.342 |
| Comparative Example 1 | 936.0 | 100 | 0 | 0.027 |
| Comparative Example 2 | 935.9 | 100 | 0 | 0.009 |
| Comparative Example 3 | 935.5 | 0 | 0 | 0.072 |
| Comparative Example 4 | 936.0 | 0 | 0 | 0 |
| Comparative Example 5 | 935.7 | 58 | 42 | 1.639 |
| Comparative Example 6 | 935.3 | 90 | 10 | 1.360 |
| Comparative Example 7 | 935.6 | 87 | 13 | 0.051 |

As seen from Tables 1 and 2, it was confirmed that the catalysts for preparation of neopentyl glycol of Example 1 to 3 exhibit superior catalytic activity as compared to the catalysts of Comparative Example 1 to 7 with Cu 2p binding energies in the range of 932 eV to 934 eV as a result of the XPS (X-ray photoelectron spectroscopy) analysis.

The catalysts for preparation of neopentyl glycol of Comparative Examples 1 and 2 showed decreased catalytic activity because the copper precursor remained unreacted under the low aging temperature condition. In addition, it can be seen that the catalysts for preparation of neopentyl glycol of Comparative Examples 3 and 4 exhibit low Cu dispersibility and show no catalytic activity although catalyst strength was increased due to crystallization of Si into quartz under the high sintering temperature condition. In addition, the catalyst for preparation of neopentyl glycol of Comparative Example 5 shows decreased catalytic activity because the fiber-based binder was used in an excess amount. In addition, the catalysts of Comparative Examples 6 and 7, wherein the fiber-based binder was not used, show decreased catalyst strength and catalytic activity.

The catalyst for preparation of neopentyl glycol according to an exemplary embodiment of the present application, which is a catalyst extrusion-molded into a cylindrical shape, is applicable to a fixed-bed reactor of a commercial scale, to which an unmolded powder catalyst is difficult to be applied, and the possibility of drifting or differential pressure caused by wearing, crushing, etc. during long-term use of the catalyst may be decreased due to superior strength and durability.

Accordingly, since the catalyst for preparation of neopentyl glycol according to an exemplary embodiment of the present application can maintain its activity even under high-temperature, high-pressure reaction conditions due to superior strength, reactivity and stability, it can increase the stability of a process for preparing neopentyl glycol and provide superior catalytic activity.

## Claims

1. A copper silicate-based catalyst comprising copper and silica, wherein
a weight ratio of Cu : Si in the copper silicate-based catalyst is 30 : 70 to 70 : 30, and
a Cu 2p binding energy of the copper silicate-based catalyst determined by XPS (X-ray photoelectron spectroscopy) analysis is 932 eV to 934 eV.

2. The copper silicate-based catalyst according to claim 1, wherein a Cu dispersibility of the copper silicate-based catalyst determined by N₂O pulse chemisorption analysis is 0.5% to 3%, and a Cu cubic crystallite size is 200 nm or smaller.

3. The copper silicate-based catalyst according to claim 1, wherein, upon NH₃-TPD (temperature-programmed desorption) analysis of the copper silicate-based catalyst, peaks are present in a weak acid site region of 100 °C or higher and lower than 400 °C and a strong acid site region of 400 °C or higher and 700 °C or lower, and a total acid amount is 0.03 mmol to 2.0 mmol.

4. The copper silicate-based catalyst according to claim 1, wherein the copper silicate-based catalyst has a crushing strength of 40 N or higher.

5. The copper silicate-based catalyst according to claim 1, wherein the copper silicate-based catalyst is a catalyst extrusion-molded into a cylindrical shape with a diameter of 2 mm to 6 mm and a height of 2 mm to 10 mm.

6. The copper silicate-based catalyst according to claim 1, wherein the copper silicate-based catalyst is for preparation of neopentyl glycol.

7. A method for preparing the copper silicate-based catalyst according to any of claims 1 to 6, comprising:
a step of preparing a coprecipitated slurry by adding a silica sol and an alkaline precipitant to an aqueous solution comprising a copper precursor;
a step of aging the coprecipitated slurry at 80 °C to 90 °C for 3 hours or longer, and then obtaining a dried product by filtering and drying the same;
a step of mixing the dried product with a fiber-based binder and an organic compound, and then preparing a catalyst precursor by molding the same; and
a step of drying the catalyst precursor, and then sintering the same at 400 °C to 650 °C, wherein
a content of the fiber-based binder is 0.1 wt% or more and less than 15 wt% based on a total weight of the dried product.

8. The method for preparing the copper silicate-based catalyst according to claim 7, wherein the alkaline precipitant comprises one or more of NH₄OH, (NH₄)₂CO₃, NH₄HCO₃, CH₄N₂O, NaOH and Na₂CO₃.

9. The method for preparing the copper silicate-based catalyst according to claim 7, wherein a content of the organic compound is 0.1 wt% to 50 wt% based on the total weight of the dried product.

10. The method for preparing the copper silicate-based catalyst according to claim 7, wherein the fiber-based binder comprises one or more of glass fiber, carbon fiber, aramid fiber, alumina fiber, aluminum silicate fiber, silicon carbide fiber and boron fiber.

11. The method for preparing the copper silicate-based catalyst according to claim 7, wherein the organic compound comprises one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

12. A method for preparing neopentyl glycol, comprising a step of conducting hydrogenation reaction by adding a hydroxypivaldehyde (HPA) solution and hydrogen to a hydrogenation reactor, wherein
the hydrogenation reactor comprises the copper silicate-based catalyst according to any of claims 1 to 6.
